(19) Europäisches Patentamt
European Patent Office
Office européen des brevets

(11) Numéro de publication: **0 215 701 B1**

(12) **FASCICULE DE BREVET EUROPEEN**

(45) Date de publication du fascicule du brevet: **25.04.90**

(51) Int. Cl.⁴: **C07C 69/743**, A01N 53/00, A61K 31/21, A23K 1/16

(21) Numéro de dépôt: **86401855.1**

(22) Date de dépôt: **21.08.86**

(54) **Nouveaux esters d'acides cyclopropane carboxyliques et de 2,3-dihydro 4-phényl 1H-inden-2-ol, leur préparation, leur application à la lutte contre les parasites et les compositions les renfermant.**

(30) Priorité: **27.08.85 FR 8512779**

(43) Date de publication de la demande: **25.03.87 Bulletin 87/13**

(45) Mention de la délivrance du brevet: **25.04.90 Bulletin 90/17**

(84) Etats contractants désignés: **CH DE GB IT LI NL**

(56) Documents cités:
**EP-A- 0 041 021**
**EP-A- 0 110 769**
**EP-A- 0 114 012**
**FR-A- 2 501 674**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris(FR)**

(72) Inventeur: **Tessier, Jean, 30, rue Jean Moulin, F-94300 Vincennes(FR)**
Inventeur: **Demoute, Jean-Pierre, 249 bis, rue de Rosny, F-93100 Montreuil Sous Bois(FR)**
Inventeur: **Babin, Didier, 22, rue de la Grenouillette, F-78180 Montigny(FR)**

(74) Mandataire: **Tonnellier, Marie-José et al, Département des Brevets ROUSSEL UCLAF B.P. no 9, F-93230 Romainville(FR)**

EP 0 215 701 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux esters d'acide cyclopropane carboxylique et de 2,3 dihydro-4-phényl-1H-inden-2-ol, leur procédé de préparation, leur application à la lutte contre les parasites et les compositions les renfermant.

On connaissait déjà certains esters de 2,3 dihydro-4-phényl-1H-inden-ol (cf FR-A 2 501 674). On vient de découvrir que de nouveaux esters de cet alcool let d'acides connus déjà utilisés dans la préparation de pesticides (cf EP-A 110 769, EP-A 114 012, EP-A 041 021) possédaient de très intéressantes propriétés biologiques.

L'invention a pour objet les composés de formule I:

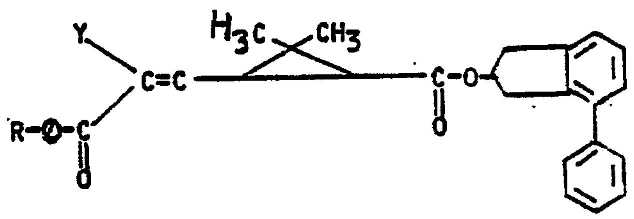

(I)

dans laquelle Y représente un atome d'hydrogène ou de fluor, R représente un radical alcoyle, linéaire, ramifié ou cyclique, renfermant de 1 à 4 atomes de carbone, la liaison éthylénique ayant la géométrie E ou Z.

Les composés de formule I peuvent exister sous de nombreuses formes stéréoisomères, ils possèdent en effet, deux carbones asymétriques en 1 et 3 du cyclopropane; ils présentent aussi une possibilité d'isomérie E/Z au niveau de la double liaison; ils possèdent également une possibilité d'isomérie (R) et (S) au niveau du carbone de la copule alcoolique en $\alpha$ du carbone du groupement $CO_2$.

Lorsque R représente un radical alcoyle, linéaire ou ramifié, il s'agit de préférence d'un radical méthyle, éthyle, n-propyle, isopropyle, n-butyle, sec-butyle, isobutyle, tert-butyle.

Lorsque R représente un radical cyclique, il s'agit de préférence d'un radical cyclopropyle ou cyclobutyle.

Parmi les composés préférés de l'invention, on peut citer les composés de formule I pour lesquels Y représente un atome d'hydrogène, la liaison éthylénique ayant la géométrie Z ainsi que ceux pour lesquels Y représente un atome de fluor, la liaison éthylénique ayant la géométrie E.

L'invention a tout spécialement pour objet les composés de formule I dans lesquels la copule cyclopropanique est de structure 1 Rcis.

L'invention a plus particulièrement pour objet les composés de formule I dans lesquels R représente un radical alcoyle linéaire, ramifié ou cyclique, renfermant de 1 à 4 atomes de carbone, comme par exemple, un radical méthyle, éthyle, isopropyle, n-butyle ou terbutyle.

L'invention a naturellement plus particulièrement pour objet les composés dont la préparation est donnée ci-après.

L'invention a également pour objet un procédé de préparation des composés de formule I caractérisé en ce que l'on soumet un acide de formule

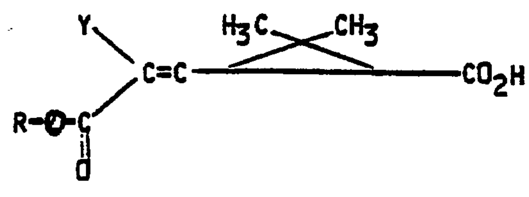

II.

dans laquelle Y et R sont définis comme précédemment, ou un dérivé fonctionnel de cet acide, à l'action de l'alcool de formule

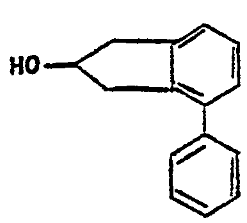

(III)

pour obtenir le composé de formule I correspondant.

Les acides de formule II sont des produits connus pouvant être préparés selon les procédés décrits

dans les brevets européens 0 038 271, 0 041 021, 0 048 186, 0 050 534 et 77 721. L'alcool de formule III est également un produit connu.

L'alcool de formule III est un produit connu qui peut être préparé selon le procédé indiqué dans le brevet américain 4 346 251.

Les composés de formule I présentent d'intéressantes propriétés qui permettent leur utilisation dans la lutte contre les parasites. Il peut s'agir, par exemple, de la lutte contre les parasites des végétaux, les parasites des locaux et les parasites des animaux à sang chaud.

C'est ainsi que l'on peut utiliser les produits de formule I pour lutter contre les insectes, les nématodes et les acariens parasites des végétaux et des animaux.

L'invention a donc pour objet les composés de formule (I) pour leur utilisation à la lutte contre les parasites des végétaux, les parasites des locaux et les parasites des animaux à sang chaud.

L'invention a donc également pour objet les compositions pesticides destinées à la lutte contre les parasites des végétaux, les parasites des locaux et les parasites des animaux à sang chaud, caractérisées en ce qu'elles renferment comme principe actif, au moins un composé défini précédemment.

Dans les compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les produits de formule (I) peuvent être utilisés notamment pour lutter contre les insectes dans le domaine agricole, pour lutter, par exemple, contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent également être utilisés pour lutter contre les insectes parasites des animaux, par exemple, les poux notamment chez les bovins, les ovins et les volailles.

L'invention a donc notamment pour objet les compositions insecticides renfermant comme principe actif au moins l'un des composés définis précédemment.

Les compositions insecticides selon l'invention contiennent de préférence de 0,005 % à 10 % en poids de matière active.

Selon un mode opératoire avantageux, pour un usage dans les locaux, les compositions insecticides selon l'invention sont utilisées sous forme de compositions fumigantes.

Les compositions insecticides selon l'invention peuvent alors être avantageusement constituées, pour la partie non active, d'un serpentin combustible ou encore d'un substrat fibreux incombustible. Dans ce dernier cas, le fumigant obtenu après incorporation de la matière active est placé sur un appareil chauffant tel qu'un électro-émanateur.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 % à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les composés de l'invention peuvent également être utilisés pour lutter contre les acariens parasites des végétaux. La partie biologique exposée ci-après met clairement en évidence les remarquables propriétés acaricides du produit de l'exemple 4.

Les composés de l'invention peuvent également être utilisés pour lutter contre les nématodes parasites des végétaux.

L'invention a donc également pour objet les compositions acaricides ainsi que les compositions nématicides renfermant comme principe actif, au moins un composé de formule I.

Les compositions acaricides et nématicides peuvent se présenter notamment sous forme de poudres, granulés, suspensions, émulsions, solutions.

Pour l'usage acaricide, on utilise de préférence des poudres mouillables, pour pulvérisation foliaire, contenant de 1 à 80 % de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Les composés de formule (I) peuvent encore être utilisés pour lutter contre les acariens parasites des animaux, pour lutter, par exemple, contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions utilisées dans la lutte contre les acariens parasites des animaux à sang chaud, caractérisées en ce qu'elles renferment au moins un produit défini ci-dessus.

Ces compositions peuvent être administrées par voie externe, par vaporisation, par shampooing, par bain ou badigeonnage. Elles peuvent être également administrées par badigeonnage de l'épine dorsale selon la méthode dite méthode "pour on". Elles peuvent être également administrées par voie digestive.

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritif peut varier selon l'espèce animale, il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des anti-oxydants.

L'invention a ainsi également pour objet les compositions destinées à l'alimentation animale renfermant comme principe actif au moins l'un des produits définis précédemment.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl)bicyclo/2,2,1/ 5-heptèn-2,3-dicarboximide, ou le pipéronyl-bis-2-(2'n-butoxy éthoxy) éthylacétal (ou tropital).

L'invention a également pour objet les associations douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cycloprane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

Les exemples suivants illustrent l'invention sans toutefois la limiter.

<u>EXEMPLE 1</u>: /1R-/1α,3α(Z)//3 -(3-méthoxy-3-oxo-1-propényl) -2,2 diméthyl cyclopropane carboxylate de 2,3 -dihydro-4-phényl -1H-inden-2-yle.

On agite pendant dix minutes, un mélange de 0,83g d'acide /1R-1α, 3α(Z)// 3-(3-méthoxy-3-oxo-1-propényl)-2,2-diméthyl cyclopropane carboxylique, 0,8 g de 2,3-dihydro-4-phényl-1H-inden -2-ol et 15 $cm^3$ de chlorure de méthylène.

On refroidit à 0°C et introduit goutte à goutte une solution renfermant 0,87 g de dicyclohexyl carbodiimide et 8 $cm^3$ de chlorure de méthylène. On laisse ensuite le mélange réactionnel obtenu sans agitation pendant 4 heures à la température ambiante.

On essore le précipité formé.

On rince à l'éther isopropylique et concentre le filtrat à sec. On obtient 1,90 g d'un produit que l'on chromatographie sur silice en éluant par le mélange hexane acétate d'éthyle 8-2. On obtient 1,37 g du produit recherché.

$\alpha_D$ = + 38°,5 + 2°,5 C = 0,5% $CHCl_3$

EXEMPLE 2: /1R-1α,3α(Z)/ 3-(3-terbutoxy-3-oxo-1-propényl) -2,2 diméthyl cyclopropane carboxylate de 2,3 dihydro-4-phényl-1H inden 2-yle.

En opérant comme précédemment, à partir de 1,01 g d'acide /1R - 1α 3α(Z)/ 3-(3-terbutoxy-3-oxo 1-propényl)-2,2-diméthyl cyclopropane carboxylique et de 0,8 g de 2,3 dihydro-4-phényl 1H inden-2-ol, on a obtenu 1,56 g de produit recherché.
$\alpha_D$ = + 46°,5 + 1°,5 (C = 1% $CHCl_3$)

EXEMPLE 3: /1R-/1α,3α,(E)// 3-(3-éthoxy-3-oxo-2-fluoro-1-propényl)-2,2 diméthyl cyclopropane carboxylate de 2,3-dihydro 4-phényl-1H-inden-2-yle.

En opérant comme à l'exemple 1, à partir de 0,97 g d'acide /1R-/1α 3α(E)// 3-(3-éthoxy-3-oxo-2-fluoro-1-propényl) 2,2-diméthyl cyclopropane carboxylique et de 0,89 g de 2,3 dihydro-4-phényl-1H-inden 2-ol, on a obtenu 1,42 g de produit recherché.
$\alpha_D$ = + 37°5 C = 0,2% $CHCl_3$

EXEMPLE 4: /1R-/1α,3α (E)/ 3-(3-terbutoxy-3-oxo-2-fluoro-1-propényl) -2,2-diméthyl cyclopropane carboxylate de 2,3-dihydro-4-phényl-1H-inden-2-yle.

En opérant comme à l'exemple 1, à partir de 1,09 g d'acide /1R-/1α 3α(E)/ 3-(3-terbutoxy-3-oxo-2-fluoro-1-propényl) -2,2-diméthyl cyclopropane carboxylique et de 0,8 g de 2,3-dihydro-4-phényl-1H-inden-2-ol, on a obtenu 1,65 g du produit recherché.
$\alpha_D$ = + 25° + 2° C = 0,7% $CHCl_3$.

**Exemples de compositions :**

**Exemple A : Préparation d'un concentré soluble**

On effectue un mélange homogène de :

| | |
|---|---|
| Produit de l'exemple 1 ................................ | 0,25 g |
| Butoxyde de pipéronyle ................................ | 1 g |
| Tween 80 ................................ | 0,25 g |
| Topanol A ................................ | 0,1 g |
| Eau ................................ | 98,4 g |

**Exemple B : Préparation d'un concentré émulsifiable**

On mélange intimement :

| | |
|---|---|
| Produit de l'exemple 4 ................................ | 0,015g |
| Butoxyde de pipéronyle ................................ | 0,5 g |
| Topanol A ................................ | 0,1 g |
| Xylène ................................ | 95,885g |
| Tween 80 ................................ | 3,5 g |

**EXEMPLE C : Préparation d'un concentré émulsifiable**

On effectue un mélange homogène de :

| | |
|---|---|
| Produit de l'exemple 4 ................................ | 1,5 g |
| Tween 80 ................................ | 20 g |
| Topanol A ................................ | 0,1 g |
| Xylène ................................ | 78,4 g |

**Etude de l'activité des composés selon l'invention sur les parasites**

Etude de l'activité des composés selon l'invention sur les parasites

Activité sur Tétranychus urticae

Essai adulticide

On utilise des plants de haricots comportant deux feuilles qui sont traitées au pistolet Fisher à différentes doses des produits à tester. Après séchage, ces plants sont infestés à raison de 25 femelles de Tétranychus urticae par feuille et maintenus à 22-23°C, 60 - 65% d'humidité relative. Les dénombrements des acariens vivants et morts sont effectués 24 heures et 48 heures après traitement.
Les produits de l'invention présentent une bonne activité adulticide dans ce test.
Les résultats sont résumés dans le tableau suivant :

| Produit de l'exemple | CL 50 en mg/hl |
| --- | --- |
| 4 | 173 |

**Revendications**

1. Les composés de formule (I):

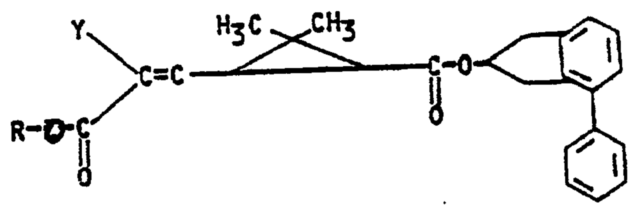

(I)

dans laquelle Y représente un atome d'hydrogène ou de fluor et R représente un radical alcoyle linéaire, ramifié ou cyclique renfermant de 1 à 4 atomes de carbone, la liaison éthylénique ayant la géométrie E ou Z.

2. Les composés de formule (I) tels que définis à la revendication 1 pour lesquels Y représente un atome d'hydrogène, la liaison éthylénique ayant la géométrie Z.

3. Les composés de formule (I) tels que définis à la revendication 1 pour lesquels Y représente un atome de fluor, la liaison éthylénique ayant la géométrie E.

4. Les composés de formule (I) tels que définis à l'une quelconque des revendications 1 à 3, dans lesquels la copule cyclopropanique est de structure 1 Rcis.

5. Les composés de formule (I) tels que définis à la revendication 4, dans lesquels R représente un radical méthyle, éthyle, isopropyle, n-butyle ou terbutyle.

6. Les composés de formule (I) tels que définis à l'une quelconque des revendications 1 à 5 dont les noms suivent:
– le [1R-[1alpha,3alpha (Z)]] 3-(3-méthoxy-3-oxo-1-propényl)-2,2-diméthyl cyclopropane carboxylate de 2,3-dihydro-4-phényl-1H-inden-2-yle.
– le [1R-[1alpha,3alpha (Z)]] 3-(3-terbutoxy-3-oxo-1-propényl)-2,2-diméthyl cyclopropane carboxylate de 2,3-dihydro-4-phényl-1H-inden-2-yle.
– le [1R-[1alpha,3alpha (E)]] 3-(3-éthoxy-3-oxo-2-fluoro-1-propényl)-2,2-diméthyl cyclopropane carboxylate de 2,3-dihydro-4-phényl-1H-inden-2-yle.
– le [1R-[1alpha,3alpha (E)]]3-(3-terbutoxy-3-oxo-2-fluoro-1-propényl)-2,2-diméthyl cyclopropane carboxylate de 2,3-dihydro-4-phényl-1H-inden-2-yle.

7. Procédé de préparation des composés de formule (I) tels que définis à l'une quelconque des revendications 1 à 6, caractérisé en ce que l'on soumet un acide de formule:

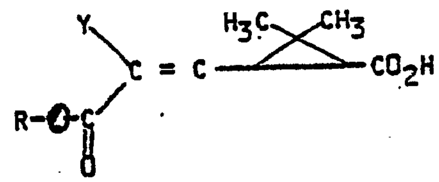

(II)

dans laquelle Y et R sont définis comme précédemment, ou un dérivé fonctionnel de cet acide, à l'action de l'alcool de formule:

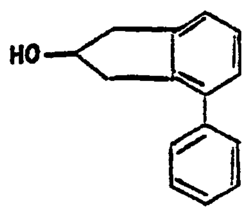

(III)

pour obtenir le composé de formule (I) correspond.

8. Les composés de formule (I) tels que définis à l'une quelconque des revendications 1 à 6 pour leur utilisation à la lutte contre les parasites des végétaux, les parasites des locaux et les parasites des animaux à sang chaud.

9. Les compositions pesticides destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 6.

10. Les compositions insecticides renfermant comme principe actif, au moins l'un des composés définis à l'une quelconque des revendications 1 à 6.

11. Les compositions acaricides destinées à lutter contre les acariens parasites des végétaux, renfermant comme principe actif au moins l'un des composés définis à l'une quelconque des revendications 1 à 6.

12. Les compositions nématicides renfermant comme principe actif, au moins l'un des composés définis à l'une quelconque des revendications 1 à 6.

13. Les compositions acaricides destinées à lutter contre les acariens parasites des animaux renfermant comme principe actif au moins l'un des composés définis à l'une quelconque des revendications 1 à 6.

14. Les compositions destinées à être incorporées à des compositions alimentaires, caractérisées en ce qu'elles renferment comme principe actif au moins l'un des composés définis à l'une quelconque des revendications 1 à 6.

## Claims

1. The compounds of formula (I):

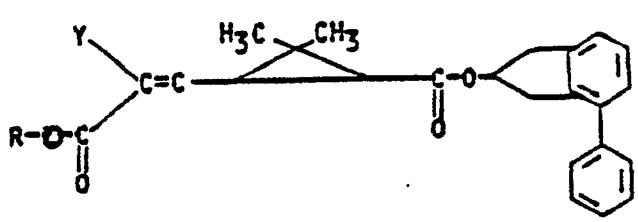

(I)

in which Y represents a hydrogen or fluorine atom and R represents a linear, branched or cyclic alkyl radical containing from 1 to 4 carbon atoms, the ethylene bond being of E or Z geometry.

2. The compounds of formula (I) as defined in claim 1 in which Y represents a hydrogen atom, the ethylene bond having Z geometry.

3. The compounds of formula (I) as defined in claim 1 in which Y represents a fluorine atom, the ethylene bond having E geometry.

4. The compounds of formula (I) as defined in any one of the claims 1 to 3, in which the cyclopropane copula is of 1 Rcis structure.

5. The compounds of formula (I) as defined in claim 4, in which R represents a methyl, ethyl, isopropyl, n-butyl or terbutyl radical.

6. The compounds of formula (I) as defined in any one of the claims 1 to 5 which have the following names:
– [1R-[1alpha,3alpha-(Z)]]-3-(3-methoxy-3-oxo-1-propenyl)-2,2-dimethyl cyclopropane carboxylate of 2,3-dihydro-4-phenyl-1H-inden-2-yl.
– [1R-[1alpha,3alpha-(Z)]]-3-(3-terbutoxy-3-oxo-1-propenyl)-2,2-dimethyl cyclopropane carboxylate of 2,3-dihydro-4-phenyl-1H-inden-2-yl.
– [1R-[1alpha,3alpha-(E)]]-3-(3-ethoxy-3-oxo-2-fluoro-1-propenyl)-2,2-dimethyl cyclopropane carboxylate of 2,3-dihydro-4-phenyl-1H-inden-2-yl.
– [1R-[1alpha,3alpha-(E)]]-3-(3-terbutoxy-3-oxo-2-fluoro-1-propenyl)-2,2-dimethyl cyclopropane carboxylate of 2,3-dihydro-4-phenyl-1H-inden-2-yl.

7. Preparation process for the compounds of formula (I) as defined in any one of claims 1 to 6, characterised in that an acid formula:

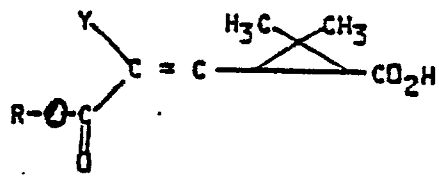

(II)

in which Y and R are defined as previously, or a functional derivative of this acid, is subjected to the action of an alcohol of formula:

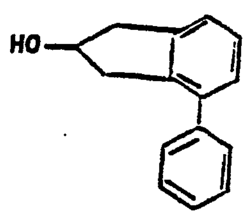

(III)

to obtain the corresponding compound of formula (I).

8. The compounds of formula (I) as defined in any one of the claims 1 to 6 for their use in combating parasites of vegetation, parasites of premises and parasites of warm-blooded animals.

9. The pesticide compositions intended for combating parasites of vegetation, parasites of premises and parasites of warm-blooded animals, characterised in that they contain as active principle at least one compound defined in any one of claims 1 to 6.

10. The insecticide compositions containing as active principle, at least one of the compounds defined in any one of claims 1 to 6.

11. The acaricide compositions intended to combat the parasitic acaridae of vegetation, containing as active principle at least one of the compounds defined in any one of claims 1 to 6.

12. The nematicide compositions containing as active principle, at least one of the compounds defined in any one of claims 1 to 6.

13. The acaricide compositions intended to combat the parasitic acaridae of animals containing as active principle at least one of the compounds defined in any one of claims 1 to 6.

14. The compositions intended to be incorporated in food stuff compositions, characterised in that they contain as active principle at least one of the compounds defined in any one of claims 1 to 6.

## Patentansprüche

1. Verbindungen der Formel (I)

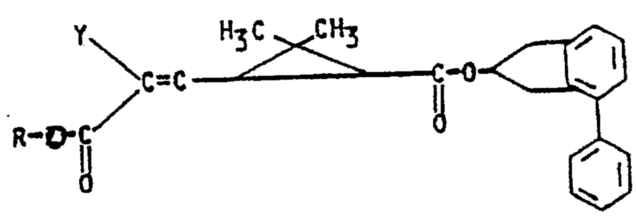

(I)

worin Y für ein Wasserstoff- oder Fluoratom steht, und R einen linearen, verzweigten oder cyclischen Alkylrest mit 1 bis 4 Kohlenstoffatomen wiedergibt, wobei die ethylenische Bindung die E- oder Z-Geometrie aufweist.

2. Verbindungen der Formel (I) gemäß Anspruch 1, worin Y ein Wasserstoffatom bedeutet, wobei die ethylenische Bindung die Z-Geometrie aufweist.

3. Verbindungen der Formel (I) gemäß Anspruch 1, worin Y ein Fluoratom bedeutet, wobei die ethylenische Bindung die E-Geometrie aufweist.

4. Verbindungen der Formel (I) gemäß einem der Ansprüche 1 bis 3, worin die Cyclopropanverknüpfung die 1-R-cis-Struktur besitzt.

5. Verbindungen der Formel (I) gemäß Anspruch 4, worin R für einen Methyl-, Ethyl-, Isopropyl-, n-Butyl- oder tert.-Butylrest steht.

6. Verbindungen der Formel (I) gemäß einem der Ansprüche 1 bis 5 mit den folgenden Bezeichnungen:

– 2,3-Dihydro-4-phenyl-1H-inden-2-yl[1R-[1α,3α(Z)]] 3-(3-methoxy-3-oxo-1-propenyl)-2,2-dimethylcyclopropan-carboxylat,

– 2,3-Dihydro-4-phenyl-1H-2-yl [1R-[1α,3α (Z)]] 3-(3-tert.-butoxy-3-oxo-1-propenyl)-2,2-dimethylcyclopropan-carboxylat,

– 2,3-Dihydro-4-phenyl-1H-inden-2-yl [1R-[1α,3α (E)]] 3-(3-ethoxy-3-oxo-2-fluor-1-propenyl)-2,2-dimethyl-cyclopropan-carboxylat,

– 2,3-Dihydro-4-phenyl-1H-inden-2-yl [1R-[1α,3α (E)]] 3-(3-tert.-butoxy-3-oxo-2-fluor-1-propenyl)-2,2-dimethyl-cyclopropan-carboxylat.

7. Verfahren zur Herstellung der Verbindungen der Formel (I) gemäß einem der Ansprüche 1 bis 6, dadurch gekennzeichnet, daß man eine Säure der Formel

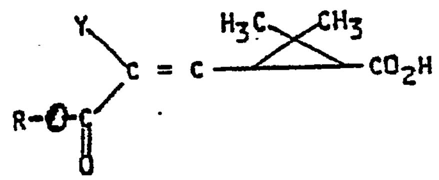

(II)

worin Y und R die vorstehende Bedeutung besitzen, oder ein funktionelles Derivat dieser Säure der Einwirkung eines Alkohols der Formel

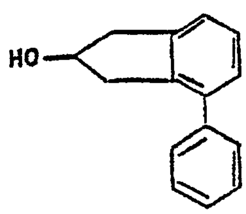

(III)

unterzieht, um zu der entsprechenden Verbindung der Formel (I) zu gelangen.

8. Verbindungen der Formel (I) gemäß einem der Ansprüche 1 bis 6 zu deren Verwendung bei der Bekämpfung von Parasiten der Pflanzen, Parasiten von Räumlichkeiten und Parasiten warmblütiger Tiere.

9. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eine Verbindung gemäß einem der Ansprüche 1 bis 6 enthalten.

10. Insektizide Zusammensetzungen, enthaltend als Wirkstoff zumindest eine der Verbindungen gemäß einem der Ansprüche 1 bis 6.

11. Akarizide Zusammensetzungen für die Bekämpfung von parasitären Milben der Pflanzen, enthaltend als Wirkstoff zumindest eine der Verbindungen gemäß einem der Ansprüche 1 bis 6.

12. Nematizide Zusammensetzungen, enthaltend als Wirkstoff zumindest eine der Verbindungen gemäß einem der Ansprüche 1 bis 6.

13. Akarizide Zusammensetzungen für die Bekämpfung von parasitären Milben der Tiere, enthaltend als Wirkstoff zumindest eine der Verbindungen gemäß einem der Ansprüche 1 bis 6.

14. Zusammensetzungen für die Einbringung in Nahrungsmittelzusammensetzungen, dadurch gekennzeichnet, daß sie als Wirkstoff zumindest eine der Verbindungen gemäß einem der Ansprüche 1 bis 6 enthalten.